# EUROPEAN PATENT APPLICATION

(11) **EP 4 410 273 A1**
(43) Date of publication of application: **07.08.2024**
(21) Application number: 21921642.1
(22) Date of filing: 16.11.2021
(51) Int. Cl.: A61K 8/73, A61K 8/04, A61K 8/02, A61K 8/92, A61Q 19/00

(54) **OIL-DISPERSED SOLID COSMETIC COMPOSITION CONTAINING HYALURONIC ACID**

(30) Priority: 29.09.2021 KR 20210128933
(71) Applicant: Cosmax, Inc., Hwaseong-si, Gyeonggi-do 18622 (KR)
(72) Inventor: KIM, Seo Yeon, Seoul 06787 (KR); LIM, Hyeon Joo, Seongnam-si, Gyeonggi-do 13523 (KR); JI, Jin Goo, Suwon-si, Gyeonggi-do 16363 (KR); PARK, Myeong Sam, Seoul 04987 (KR)
(74) Representative: Plougmann Vingtoft a/s
(86) International application number: PCT/KR2021/016696
(87) International publication number: WO 2023/054802

(57) **Abstract**

The present invention relates to an oil dispersion solid cosmetic composition containing a hyaluronic acid, wherein the oil dispersion solid cosmetic composition can implement a stable formulation by the uniform dispersion of the hyaluronic acid as a water-soluble ingredient in an oily phase and has an improved effect in skin moisturization by containing the hyaluronic acid.

## Description

### Technical Field

This application claims priority to Korean Patent Application No. 10-2021-0128933 filed in the Korean Intellectual Property Office on 29 September 2021, the disclosure of which is incorporated herein by reference.

The present disclosure relates to an oil dispersion solid cosmetic composition having an improved moisturizing effect by containing a hyaluronic acid.

### Background Art

The disruption of the oil and moisture balance makes the skin drier or increases the amount of sebum secretion, thereby worsening the skin condition, so it is very important to control the oil and moisture balance in the skin. In particular, the drying of the skin due to a lack of moisture easily causes the exfoliation of dead skin cells, damage to the skin by an external stimulation, and skin troubles.

Hyaluronic acid is a component that is originally present in the skin. As the amount of hyaluronic acid decreases with aging, the skin becomes dry and develops fine wrinkles. Hyaluronic acid has the ability to attract and store moisture 200 times the size thereof, exert an excellent action of replenishing moisture due to the presence in the subcutaneous tissue, and is known to help in maintaining youthful skin. Recently, applicable hyaluronic acid cosmetics or edible hyaluronic acid nutritional supplements like vitamins are being developed.

Conventional oil dispersion solid cosmetic compositions contain a high-content oil mixed therein, and thus have the advantage of helping moisturize the skin by forming an oil film when applied to the skin. However, in the oil dispersion solid cosmetic compositions containing a high-content oil, the mixing of water-soluble raw materials is not easy, and thus the mixing of water-soluble raw materials adversely affects formulation stability.

Therefore, there is an urgent need to develop an oil dispersion solid cosmetic composition, to which a hyaluronic acid as a water-soluble ingredient is applied and in which the water-soluble ingredient is uniformly dispersed.

### Disclosure of Invention

### Technical Problem

The present inventors have made intensive research efforts to develop a stable oil dispersion solid cosmetic composition containing a water-soluble raw material mixed therein. As a result, the present inventors confirmed that an oil dispersion solid cosmetic composition containing a hyaluronic acid implements a stable formulation and has an improved effect in skin moisturization.

Accordingly, an aspect of the present disclosure is to provide an oil dispersion solid cosmetic composition containing a hyaluronic acid, an oil, a wax, and a butter.

### Solution to Problem

Hereinafter, the present disclosure will be described in more detail.

An aspect of the present disclosure is directed to an oil dispersion solid cosmetic composition containing a hyaluronic acid, an oil, a wax, and a butter.

In the present disclosure, the hyaluronic acid may be at least one selected from the group consisting of sodium hyaluronate, hydrolyzed hyaluronic acid, hydroxypropyltrimonium hyaluronate, hyaluronic acid, and sodium acetylated hyaluronate, but is not limited thereto.

In the present disclosure, the hyaluronic acid may be contained, relative to the total cosmetic composition, in a content of 0.01 to 1.0 wt%, 0.01 to 0.8 wt%, 0.01 to 0.6 wt%, 0.1 to 1.0 wt%, 0.1 to 0.8 wt%, 0.1 to 0.6 wt%, 0.3 to 1.0 wt%, 0.3 to 0.8 wt%, or 0.3 to 0.6 wt%, and for example, 0.6 wt%, but is not limited thereto.

In the present disclosure, the oil may be at least one selected from the group consisting of polyglyceryl-2 triisostearate, sunflower seed oil, Cocos *nucifera* (coconut) oil, sweet almond oil, *Pyrus malus* (apple) seed oil, hydrogenated castor oil, meadowfoam seed oil, triethylhexanoin, diisostearyl malate, olive oil, castor oil, avocado oil, armond oil, rosehip oil, macadamia nut oil, mink oil, liquid paraffin/paraffin oil, Vaseline, myristic acid isopropyl, silicone oil, jojoba oil, green tea oil, grape seed oil, argan oil, and hydrogenated polydecene, but is not limited thereto.

In the present disclosure, the oil may be contained, relative to the total cosmetic composition, in a content of 50.0 to 90.0 wt%, 50.0 to 85.0 wt%, 50.0 to 80.0 wt%, 55.0 to 90.0 wt%, 55.0 to 85.0 wt%, 55.0 to 80.0 wt % , 60.0 to 90.0 wt % , 60.0 to 85.0 wt % , 60.0 to 80.0 wt % , 65.0 to 90.0 wt % , 65.0 to 85.0 wt % , 65.0 to 80.0 wt%, 70.0 to 90.0 wt%, or 70.0 to 85.0 wt%, and for example, 70.0 to 80.0 wt%, but is not limited thereto.

In the present disclosure, the wax may be at least one selected from the group consisting of candelilla wax, microcrystalline wax, octyldodecanol, bees wax, *Copernicia cerifera* (carnauba) wax, cerecin, synthetic wax, synthetic beeswax, and ozokerite, but is not limited thereto.

In the present disclosure, the wax may be contained, relative to the total cosmetic composition, in a content of 5.0 to 40.0 wt%, 5.0 to 35.0 wt%, 5.0 to 30.0 wt % , 5.0 to 25.0 wt % , 5.0 to 20.0 wt % , 10.0 to 40.0 wt % , 10.0 to 35.0 wt % , 10.0 to 30.0 wt % , 10.0 to 25.0 wt % , 10.0 to 20.0 wt % , 15.0 to 40.0 wt % , 15.0 to 35.0 wt % , 15.0 to 30.0 wt % , 15.0 to 25.0 wt%, or 15.0 to 20.0 wt%, and for example, 20.0 wt%, but is not limited thereto.

In the present disclosure, the butter may be at least one selected from the group consisting of shea butter, *Mangifera indica* (mango) seed butter, *Theobroma cacao* (cocoa) seed butter, cupuacu seed butter, *Cocos nucifera* (coconut) seed butter, *Shorea robusta* seed butter, and *Garcinia indica* seed butter, but is not limited thereto.

In the present disclosure, the butter may be contained, relative to the total cosmetic composition, in a content of 2.0 to 10.0 wt%, 2.0 to 8.0 wt%, 2.0 to 6.0 wt%, 4.0 to 10.0 wt%, 4.0 to 8.0 wt%, or 4.0 to 6.0 wt%, and for example, 5.0 wt%, but is not limited thereto.

In the present disclosure, the oil dispersion solid cosmetic composition may further contain an antioxidant and a preservative, but is not limited thereto.

### Advantageous Effects of Invention

The present disclosure relates to an oil dispersion solid cosmetic composition containing a hyaluronic acid, wherein the oil dispersion solid cosmetic composition can implement a stable formulation by the uniform dispersion of the hyaluronic acid as a water-soluble ingredient in an oily phase and has an improved effect in skin moisturization by containing the hyaluronic acid.

### Brief Description of Drawings

FIG. 1 is an image showing formulations of Examples 1 and 2 and Comparative Example 2 according to one test example of the present disclosure.
FIG. 2 is a graph showing the evaluation results of skin moisturizing effect of Examples 1 and 2 and Comparative Example 2 according to one test example of the present disclosure.

### Best Mode for Carrying out the Invention

The present disclosure relates to an oil dispersion solid cosmetic composition containing a hyaluronic acid, an oil, a wax, and a butter.

### Mode for Carrying out the Invention

Hereinafter, the present disclosure will be described in more detail by the following examples. However, these exemplary embodiments are used only for illustration, and the scope of the present disclosure is not limited by these exemplary embodiments.

### Preparative Example: Preparation of Examples and Comparative Examples

Oil dispersion solid cosmetic compositions of Example 1 and Comparative Example 1 were prepared according to each ingredient and content thereof shown in Table 1 below.

**TABLE 1**

| No | Classification | Ingredient | Example 1 | Example 2 | Comparative Example 1 | Comparative Example 2 |
|---|---|---|---|---|---|---|
| 1 | Hyaluronic acid | Sodium hyaluronate | 0.6 | 0.001 | 0.6 | 0.6 |
| 2 | Wax | Candelilla wax | 15.0 | 15.0 | 2.5 | 40.0 |
| 3 | | Microcrystalline wax | 1.7 | 1.7 | 0.5 | 2.0 |
| 4 | | Octyldodecanol | 3.0 | 3.0 | - | 3.0 |
| 5 | Oil | Polyglyceryl-2 triisostearate | 30.0 | 30.0 | 30.0 | 30.0 |
| 6 | | Sunflower seed oil | 33.0 | 33.599 | 49.7 | 7.7 |
| 7 | | Sweet almond oil | 5.0 | 5.0 | 5.0 | 5.0 |
| 8 | | Apple seed oil | 5.0 | 5.0 | 5.0 | 5.0 |
| 9 | | Hydrogenated castor oil | 0.3 | 0.3 | 0.3 | 0.3 |
| 10 | | Meadowfoam seed oil | 1.4 | 1.4 | 1.4 | 1.4 |
| 12 | Butter | Shea butter | 5.0 | 5.0 | 5.0 | 5.0 |
| Total (unit: wt%) | | | 100.0 | 100.0 | 100.0 | 100.0 |

Specifically, all raw materials were weighed in beakers according to each ingredient and content thereof shown in Table 1, and then uniformly mixed at 25 rpm for 5 minutes by an Agi-Mixer while warmed at 75 to 85°C. Then, the mixtures were defoamed, poured into stick containers, and cooled, thereby preparing stick type cosmetic compositions.

### Test Example 1: Evaluation of formulation stability

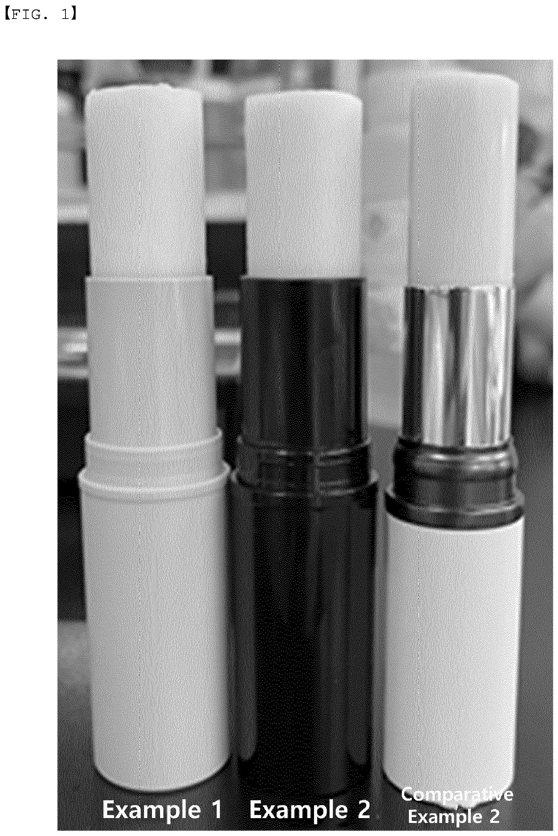

Examples 1 and 2 and Comparative Examples 1 and 2 were investigated for formulation stability. Specifically, each cosmetic composition was placed under conditions of 4°C, room temperature, 37°C, 45°C, and light for 1 day, 5 days, 1 week, 2 weeks, and 4 weeks, and then evaluated for formulation stability. The results are shown in Table 2 and FIG. 1. The evaluation criteria were as follows.

### <Evaluation Criteria>

Stable; ⊚
Slight unstable; △
Unstable; ×

**TABLE 2**

| | | Example 1 | Example 2 | Comparative Example 2 |
|---|---|---|---|---|
| 1 Day | 4°C | ⊚ | ⊚ | ⊚ |
| | Room temperature | ⊚ | ⊚ | ⊚ |
| | 37°C | ⊚ | ⊚ | ⊚ |
| | 4 5°C | ⊚ | ⊚ | ⊚ |
| | Light | ⊚ | ⊚ | ⊚ |
| 5 Days | 4 °C | ⊚ | ⊚ | ⊚ |
| | Room temperature | ⊚ | ⊚ | ⊚ |
| | 37°C | ⊚ | ⊚ | ⊚ |
| | 4 5°C | ⊚ | ⊚ | ⊚ |
| | Light | ⊚ | ⊚ | ⊚ |
| 1 week | 4°C | ⊚ | ⊚ | ⊚ |
| | Room temperature | ⊚ | ⊚ | ⊚ |
| | 37°C | ⊚ | ⊚ | ⊚ |
| | 4 5°C | ⊚ | ⊚ | ⊚ |
| | Light | ⊚ | ⊚ | ⊚ |
| 2 Weeks | 4°C | ⊚ | ⊚ | ⊚ |
| | Room temperature | ⊚ | ⊚ | ⊚ |
| | 37°C | ⊚ | ⊚ | ⊚ |
| | 4 5°C | ⊚ | ⊚ | ⊚ |
| | Light | ⊚ | ⊚ | ⊚ |
| 4 Weeks | 4°C | ⊚ | ⊚ | ⊚ |
| | Room temperature | ⊚ | ⊚ | ⊚ |
| | 37°C | ⊚ | ⊚ | ⊚ |
| | 4 5°C | ⊚ | ⊚ | ⊚ |
| | Light | ⊚ | ⊚ | ⊚ |

As can confirmed in Table 2 and FIG. 1, Examples 1 and 2 and Comparative Example 2 showed a stable formulation even 4 weeks after preparation. However, Comparative Example 1 was excluded from the observation of stability since a stick formulation could not be formed. Comparative Example 2 showed a stable formulation, but due to a wax content exceeding 40 wt%, the formulation hardness was high and the spreadability greatly deteriorated.

### Test Example 2: Evaluation of skin moisturizing

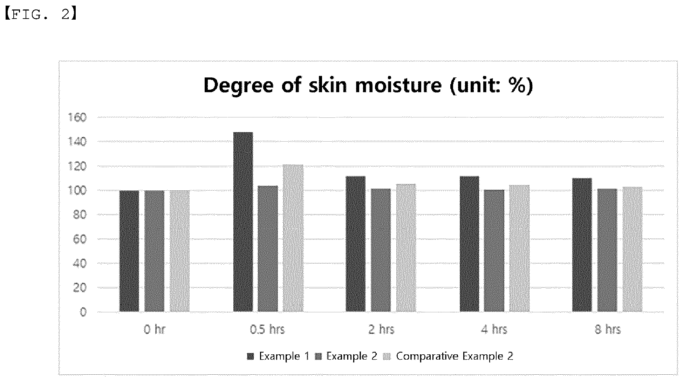

To evaluate the skin moisturizing effect of Examples 1 and 2 and Comparative Example 2, skin moisturization evaluation was performed on 20 subjects aged 25 to 25 years. To measure the amount of moisture present in the skin epidermis, the Corneometer (CK electronic, Germany) sensor, which is an instrument capable of checking skin moisturizing ability by measuring and quantifying the ion degree of moisture, was used. The degree of skin moisture was expressed as %, and measured under conditions of a constant temperature of 22±2°C and a constant humidity (relative humidity) of 40 to 60%. The results are shown in Table 3 and FIG. 2.

**TABLE 3**

| (Unit: %, degree of skin moisture) | Example 1 | Example 2 | Comparative Example 2 |
|---|---|---|---|
| 0 hours | 100.0 | 100.0 | 100.0 |
| 0.5 hours | 148.3 | 104.2 | 121.5 |
| 2 hours | 111.8 | 101.7 | 105.7 |
| 4 hours | 112.3 | 100.8 | 104.8 |
| 8 hours | 110.1 | 101.3 | 103.5 |

As can be confirmed in Table 3 and FIG. 2, the degree of skin moisture was 148.3% for 0.5 hours, 111.8% for 2hours, 112.3% for 4 hours, and 110.1% for 8 hours in Example 1, indicating that Example 1 showed a higher degree of skin moisture than Example 2 and Comparative Example 2 at all the time periods.

It can be therefore confirmed that Example 1 has an excellent in skin moisturizing effect.

### Test Example 3: Sensory evaluation

Examples 1 and 2 and Comparative Example 2 were applied to the skin of 20 male/female panels aged 20 to 40 years, and then evaluated for a feeling in use upon application, such as spreadability, a glossy appearance, absorbing ability, a moisturizing feeling, and overall satisfaction, according to the following evaluation criteria. The results are shown in Table 4.

In the criteria, the spreadability refers to a degree at which a composition is smoothly spread with no brake, with respect to a feeling of being spread on the skin. The glossy appearance refers to a degree of skin glossiness immediately after application and 10 minutes after application. The absorbing ability refers to a degree at which a composition is absorbed into the skin 10 minutes after application to the skin. The moisturizing feeling refers to a degree at which the skin feels moist without feeling sticky, damp, or greasy by a composition 10 minutes after skin application. These four criteria are used to increase the value of use as a cosmetic material, and especially, the absorbing ability and moisture feeling may be important characteristics of an oil dispersion solid cosmetic composition.

### <Evaluation Criteria>

⊚; 15 or more users determining a composition as being excellent
o; 10 to 14 users determining a composition as being excellent
△; 9 or less users determining a composition as being excellent

**TABLE 4**

| | Spreadability | Glossy appearance | Absorbing ability | Moisturizing feeling | Overall satisfaction |
|---|---|---|---|---|---|
| Example 1 | ⊚ | ⊚ | ○ | ⊚ | ⊚ |
| Example 2 | ⊚ | ⊚ | ○ | Δ | ○ |
| Comparative Example 2 | Δ | Δ | ○ | Δ | Δ |

As can be confirmed in Table 4 above, Example 2 was evaluated to be lower than Example 1 in terms of a moisturizing feeling. Comparative Example 2, compared with Example 1, was relatively hard and thus caused poor spreadability, resulting in deterioration in terms of a glossy appearance and moisturizing ability. However, Example 1 received a high evaluation in terms of each of spreadability, a glossy appearance, and a moisturizing feeling, and received the highest evaluation in terms of overall satisfaction.

### Industrial Applicability

The present disclosure relates to an oil dispersion solid cosmetic composition having an improved moisturizing effect by containing a hyaluronic acid.

## Claims

1. An oil dispersion solid cosmetic composition comprising a hyaluronic acid, an oil, a wax, and a butter.

2. The oil dispersion solid cosmetic composition of claim 1, wherein the hyaluronic acid is at least one selected from the group consisting of sodium hyaluronate, hydrolyzed hyaluronic acid, hydroxypropyltrimonium hyaluronate, hyaluronic acid, and sodium acetylated hyaluronate.

3. The oil dispersion solid cosmetic composition of claim 1, wherein the hyaluronic acid is contained in a content of 0.01 to 1.0 wt% relative to the total cosmetic composition.

4. The oil dispersion solid cosmetic composition of claim 1, wherein the oil is at least one selected from the group consisting of polyglyceryl-2 triisostearate, sunflower seed oil, *Cocos nucifera* (coconut) oil, sweet almond oil, *Pyrus malus* (apple) seed oil, hydrogenated castor oil, meadowfoam seed oil, triethylhexanoin, diisostearyl malate, olive oil, castor oil, avocado oil, armond oil, rosehip oil, macadamia nut oil, mink oil, liquid paraffin/paraffin oil, Vaseline, myristic acid isopropyl, silicone oil, jojoba oil, green tea oil, grape seed oil, argan oil, and hydrogenated polydecene.

5. The oil dispersion solid cosmetic composition of claim 1, wherein the oil is contained in a content of 50.0 to 90.0 wt% relative to the total cosmetic composition.

6. The oil dispersion solid cosmetic composition of claim 1, wherein the wax is at least one selected from the group consisting of candelilla wax, microcrystalline wax, octyldodecanol, bees wax, *Copernicia cerifera* (carnauba) wax, cerecin, synthetic wax, synthetic beeswax, and ozokerite.

7. The oil dispersion solid cosmetic composition of claim 1, wherein the wax is contained in a content of 5.0 to 40.0 wt% relative to the total cosmetic composition.

8. The oil dispersion solid cosmetic composition of claim 1, wherein the butter is at least one selected from the group consisting of shea butter, *Mangifera indica* (mango) seed butter, *Theobroma cacao* (cocoa) seed butter, cupuacu seed butter, *Cocos nucifera* (coconut) seed butter, *Shorea robusta* seed butter, and *Garcinia indica* seed butter.

9. The oil dispersion solid cosmetic composition of claim 1, wherein the butter is contained in a content of 2.0 to 10.0 wt% relative to the total cosmetic composition.

10. The oil dispersion solid cosmetic composition of claim 1, further comprising an antioxidant and a preservative.
